# EUROPEAN PATENT SPECIFICATION

(11) **EP 1 294 334 B1**
(45) Date of publication and mention of the grant of the patent: **04.04.2007**
(21) Application number: 01939763.7
(22) Date of filing: 31.05.2001
(51) Int. Cl.: A61F 13/15, B29C 65/02, B29C 65/08, D04H 13/00, B32B 5/26

(54) **LEAK-PROOF ULTRASONIC POINT-BONDS**
FLÜSSIGKEITSUNDURCHLÄSSIGE ULTRASCHALLERZEUGTE PUNKTVERSCHWEISSUNGEN
POINTS D'ADHERENCE INTERMITTENTS ANTI-FUITE CREES PAR ULTRASONS

(30) Priority: 01.06.2000 US 209393 P; 04.05.2001 US 849594
(43) Date of publication of application: 26.03.2003
(73) Proprietor: KIMBERLY-CLARK WORLDWIDE, INC., Neenah, WI 54956 (US)
(72) Inventor: SORENSEN, Daniel, J., Neenah, WI 54956 (US); POPP, Robert, Lee, Hortonville, WI 54944 (US)
(74) Representative: Davies, Christopher Robert
(86) International application number: PCT/US2001/017605
(87) International publication number: WO 2001/091992

(56) References cited:
- EP-A- 0 677 284
- EP-A- 0 689 930
- GB-A- 2 257 652
- US-A- 3 303 084
- US-A- 4 572 753
- US-A- 4 767 492
- US-A- 5 624 420
- US-A- 5 817 199

## Description

### FIELD OF THE INVENTION

This invention relates to intermittent ultrasonic bonds that create a leak-proof seal.

### BACKGROUND OF THE INVENTION

Absorbent garments, including disposable diapers and training pants, and other personal care products require leak-proof seals in their assembly to ensure proper containment of bodily wastes, for example. Such leak-proof seals are typically accomplished through a continuous, or solid, bond pattern. This continuous pattern is usually formed by hot melt adhesives, thermal bonds or ultrasonic bonds.

Ultrasonic bond patterns can be intermittent points, applied in a pattern, that create a thermal bond between two or more layers of material. The bonds are normally stronger when a pattern of individual points is used rather than a continuous, or solid, bond. However, because intermittent bond points are spaced apart from one another, fluids can pass between the bond points and, therefore, the intermittent bonds are typically not leak-proof, or moisture proof.

There is a need or desire for a strong, leak-proof seal that can be used in the assembly of absorbent garments and other personal care products.

### SUMMARY OF THE INVENTION

The present invention is directed to intermittent ultrasonic bonds that create a strong, leak-proof seal. These bonds are particularly suitable for use in the assembly of absorbent garments and personal care products, including disposable diapers, training pants, feminine hygiene products, adult incontinence products, and medical garments. A strong, leak-proof seal is especially desirable for attaching containment flaps to garments, and for bonding composite structures.

The leak-proof, or moisture-proof, seal is created when at least two layers of material are ultrasonically bonded with numerous point bonds that are spaced sufficiently close together to create a seal between the bonds. When ultrasonic bonds are used to form a bond, part of the substrate material being bonded is displaced outside of the actual bond point. In this invention, the displaced materials either contact each other, thereby blocking any passage of fluid between the bond points, or are close enough together to create a sufficiently tortuous path to block any passage of fluid between the bond points. Furthermore, the strength of the bond is optimized through the use of point bonds, versus a solid bond.

With the foregoing in mind, it is a feature and advantage of the invention to provide a strong, leak-proof seal.

It is also a feature and advantage of the invention to provide a bonding process for bonding together at least two layers of material resulting in a strong, leak-proof seal.

These and other features and advantages will become further apparent from the following detailed description of the presently preferred embodiments, read in conjunction with the drawings.

### BRIEF DESCRIPTION OF THE DRAWINGS

Fig. 1 illustrates an enlarged, partial plan view of one type of point bond pattern bonding two sheets of material together in accordance with the present invention; and
Fig. 2 illustrates an enlarged sectional view of intermittent ultrasonic bond points bonding two sheets of material together, taken along line 2-2 of Fig. 1.

### DEFINITIONS

Within the context of this specification, each term or phrase below will include the following meaning or meanings.

"Bond point" refers to a discrete point where ultrasonic bonding has occurred.

"Bonded" refers to the joining, adhering, connecting, attaching, or the like, of two elements. Two elements will be considered to be bonded together when they are bonded directly to one another or indirectly to one another, such as when each is directly bonded to intermediate elements.

"Breathable" describes materials that typically block the passage of particulate matter, water and other liquids while allowing water vapor and air to pass through the material.

"Disposable" refers to articles which are designed to be discarded after a limited use rather than being laundered or otherwise restored for reuse.

"Elastic," "elasticized" and "elasticity" mean that property of a material or composite by virtue of which it tends to recover its original size and shape after removal of a force causing a deformation.

"Film" refers to a thermoplastic film made using a film extrusion and/or foaming process, such as a cast film or blown film extrusion process. The term includes apertured films, slit films, and other porous films which constitute liquid transfer films, as well as films which do not transfer liquid.

"Layer" when used in the singular can have the dual meaning of a single element or a plurality of elements.

"Liquid impermeable," when used in describing a layer or multi-layer laminate, means that a liquid, such as urine, will not pass completely through the layer or laminate, under ordinary use conditions, in a direction generally perpendicular to the plane of the layer or laminate at the point of liquid contact. Liquid, or urine, may spread or be transported parallel to the plane of the liquid impermeable layer or laminate, so long as it does not pass through the layer or laminate.

"Liquid permeable material" or "liquid water-permeable material" refers to a material present in one or more layers, such as a film, nonwoven fabric, or open-celled foam, which is porous, and which is water permeable due to the flow of water and other aqueous liquids through the pores. The pores in the film or foam, or spaces between fibers or filaments in a nonwoven web, are large enough and frequent enough to permit leakage and flow of liquid water through the material.

"Medical garment" refers to medical (i.e., protective and/or surgical) gowns, caps, gloves, drapes, face masks, and the like.

"Meltblown fiber" means fibers formed by extruding a molten thermoplastic material through a plurality of fine, usually circular, die capillaries as molten threads or filaments into converging high velocity heated gas (e.g., air) streams which attenuate the filaments of molten thermoplastic material to reduce their diameter, which may be to microfiber diameter. Thereafter, the meltblown fibers are carried by the high velocity gas stream and are deposited on a collecting surface to form a web of randomly dispersed meltblown fibers. Such a process is disclosed for example, in U.S. Patent 3,849,241 to Butin et al. Meltblown fibers are microfibers which may be continuous or discontinuous, are generally smaller than about 0.6 denier, and are generally self bonding when deposited onto a collecting surface. Meltblown fibers used in the present invention are preferably substantially continuous in length.

"Nonwoven" and "nonwoven web" refer to a material or a web of material having a structure of individual fibers or filaments which are interlaid, but not in an identifiable manner as in a knitted fabric. The terms "fiber" and "filament" are used herein interchangeably. Nonwoven fabrics or webs have been formed from many processes such as, for example, meltblowing processes, spunbonding processes, air laying processes, and bonded carded web processes. The term also includes films that have been perforated or otherwise treated to allow air to pass through.

"Offset" refers to an angle measured from a point on a line to another point not on the line.

"Point bond" refers to a type of bond that is discontinuous, as opposed to a continuous, or solid, bond.

"Polymers" include, but are not limited to, homopolymers, copolymers, such as for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to isotactic, syndiotactic and atactic symmetries.

"Spunbonded fiber" refers to small diameter fibers which are formed by extruding molten thermoplastic material as filaments from a plurality of fine capillaries of a spinnerette having a circular or other configuration, with the diameter of the extruded filaments then being rapidly reduced as by, for example, in U.S. Patent 4,340,563 to Appel et al., and U.S. Patent 3,692,618 to Dorschner et al., U.S. Patent 3,802,817 to Matsuki et al., U.S. Patents 3,338,992 and 3,341,394 to Kinney, U.S. Patent 3,502,763 to Hartmann, U.S. Patent 3,502,538 to Petersen, and U.S. Patent 3,542,615 to Dobo et al.. Spunbond fibers are quenched and generally not tacky when they are deposited onto a collecting surface. Spunbond fibers are generally continuous and often have average deniers larger than about 0.3, more particularly, between about 0.6 and 10.

"Stretchable" means that a material can be stretched, without breaking, to at least 150% of its initial (unstretched) length in at least one direction, suitably to at least 200% of its initial length, desirably to at least 250% of its initial length.

"Thermoplastic" describes a material that softens when exposed to heat and which substantially returns to a nonsoftened condition when cooled to room temperature.

These terms may be defined with additional language in the remaining portions of the specification.

### DETAILED DESCRIPTION OF THE PRESENTLY PREFERRED EMBODIMENTS

The principles of this invention can be applied to a wide variety of garments and personal care products, including disposable garments having at least two layers 12, 14 of material bonded together wherein the resulting seam is exposed to moisture during routine use. Examples include diapers, training pants, certain feminine hygiene products, adult incontinence products, other personal care or medical garments, and the like. More particularly, this invention can be applied to the attachment of containment flaps to such garments and to the bonding of composite structures.

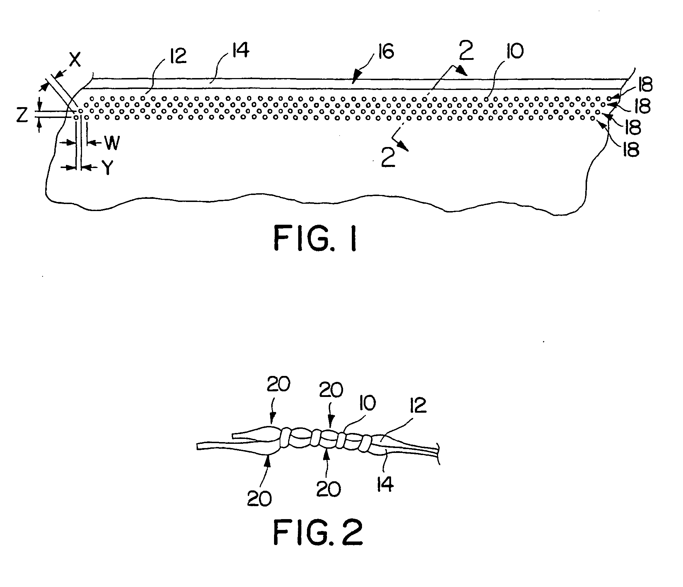

Referring to Fig. 1, a pattern of intermittent ultrasonic bond points 10 is shown applied to two layers 12, 14 of material, wherein a first layer 12 is positioned on top of a second layer 14 and the second layer 14 extends beyond the first layer 12. The first layer 12 and the second layer 14 must at least partially overlap. The bond points 10 bond the two layers 12, 14 together, resulting in a strong, leak-proof seal 16. The seal 16 is strong due to the many bond points 10, and small distances between the bond points 10 prevent moisture from penetrating through the seal 16. As shown in Fig. 1, one embodiment of the invention includes multiple parallel rows 18 of bond points 10, wherein the bond points 10 in adjacent rows 18 are offset from one another, anywhere from about 0 degrees to about 89 degrees, alternatively from about 15 degrees to about 75 degrees, as yet another alternative, from about 30 degrees to about 60 degrees. This invention requires at least one row 18 of bond points 10, or at least two rows 18 of bond points 10, alternatively at least three rows 18, or, as another alternative, at least four rows 18.

Each bond point 10 is suitably round, or any other suitable shape such as oblong, figure-eight, or rectangular, for example. Each bond point 10 suitably has a diameter of between about 0.005 inch (0.013 cm) and about 0.25 inch (0.64 cm), alternatively between about 0.010 inch (0.025 cm) and about 0.175 inch (0.44 cm), or, as another alternative, between about 0.015 inch (0.038 cm) and about 0.15 inch (0.38 cm).

Similarly, each bond point 10 is a distance of between about 0.001 inch (0.0025 cm) and about 0.20 inch (0.51 cm) away from at least one other bond point 10 in either a machine direction or a cross direction, or any direction therebetween. Alternatively, each bond point 10 is within about 0.0025 inch (0.0064 cm) to about 0.175 inch (0.44 cm) of at least one other bond point 10. As another alternative, each bond point 10 is within about 0.005 inch (0.013 cm) to about 0.15 inch (0.38 cm) of at least one other bond point 10.

Examples of various measurable distances between the bond points 10 are shown in Fig. 1. More specifically, a distance W is a linear distance between two adjacent bond points 10 in a row 18, a distance X is a linear distance between two bond points 10 diagonal from one another, a distance Y is a horizontal distance between the two diagonal bond points 10 wherein the two points 10 are vertically offset, and a distance Z is a vertical distance between two bond points 10 wherein the two bond points 10 are horizontally offset. The pattern width is the total vertical distance between the top row 18 and the bottom row 18 of bond points 10.

When ultrasonic bond points 10 are applied to a substrate 12, part of the substrate material being bonded is displaced outside of the actual bond point 10, as shown in Fig. 2. In this invention, the displaced materials 20 of each layer 12, 14 either contact each other, or are close enough together to create a sufficiently tortuous path to block any passage of fluid between the bond points 10. Due to the heat generated by the ultrasonic bonding process at the point bonds 10, the displaced materials 20 between the point bonds 10 are in a molten form when the displacement occurs. The molten displaced materials 20 bond together when in contact with one another due to the heat generated and pressure exerted during the ultrasonic bonding process, thereby forming a light bond between the displaced materials 20 of adjacent layers 12, 14 and blocking any passage of fluid between the bond points 10 and the displaced materials 20 between the layers 12,14. Furthermore, the strength of the seal 16 is optimized through the use of point bonds, versus a solid bond.

The ultrasonic bonding process can be carried out by a variety of methods (not shown), including using an ultrasonic stationary horn or an ultrasonic rotary horn or different frequency horns. In the stationary method, a stationary anvil is located below the ultrasonic horn, or wave guide. The materials 12, 14 are coupled to the horn, or wave guide, such that the materials 12, 14 are between the horn and the anvil. The horn vibrates at a specific rate, e.g. 20,000 cycles/sec, with an amplitude of roughly 0.0005 inch (0.0013 cm) to about 0.0035 inch (0.0089 cm) emitting ultrasonic energy through friction and thereby bonding the materials 12, 14 together.

In the rotary method, the materials 12, 14 are placed between a rotating disk and a rotating anvil, preferably a circular anvil with a pattern on a surface contacting the materials 12, 14. The rotating disk vibrates, expanding and contracting around its circumference by up to about 0.0035 inch (0.0089 cm). As in the stationary method, friction causes the emission of ultrasonic energy, thereby bonding the materials 12, 14 together. Furthermore, the pattern on the anvil forms a bonding pattern that is transferred to the materials 12, 14.

Suitable substrate materials for the at least two layers 12, 14 of material in the present invention include liquid-impermeable materials, including nonwoven web laminates made from a wide selection of materials. Various nonwoven fabrics can be laminated to a film or other liquid-impermeable layer to be used as the substrate. For example, the substrate can be composed of a meltblown or spunbonded web of polyolefin fibers, laminated to a liquid impermeable layer. The substrate can also be a bonded-carded web composed of natural and/or synthetic fibers, laminated to a liquid impermeable layer. While the layers 12, 14 are suitably liquid-impermeable, the layers 12, 14 may be breathable as well.

The substrate is preferably a liquid impermeable material or is laminated to a liquid impermeable material. One example of a suitable liquid impermeable material is a thin plastic film, although other flexible liquid impermeable materials may also be used. A suitable liquid impermeable film for use as a substrate is a polyolefin film, for instance, a 0.2 millimeter polyethylene film commercially available from Huntsman Packaging of Newport News, Virginia, U.S.A.

The substrate can also include an elastic material, for example, as described in terms of side panels of an absorbent garment in U.S. Patent No. 4,940,464, issued July 10, 1990, to Van Gompel et al., which is incorporated herein by reference. Furthermore, the elastic material can be used as virtually any part of an absorbent garment or personal care product and can include a stretch-thermal laminate (STL), a neck-bonded laminated (NBL), a reversibly necked laminate, or a stretch-bonded laminate (SBL) material. Methods of making such materials are well known to those skilled in the art and described in U.S. Patent 4,663,220 issued May 5, 1987 to Wisneski et al.; U.S. Patent 5,226,992 issued July 13, 1993 to Morman; and European Patent Application No. EP 0 217 032 published on April 8, 1957 in the names of Taylor et al.. Alternatively, these elastic materials may include other woven or nonwoven materials, or stretchable but inelastic materials.

As mentioned, the present invention can be applied to diapers, training pants, certain feminine hygiene products, adult incontinence products, other personal care or medical garments, and the like.

### EXAMPLE

Nine different bond patterns were produced and evaluated for their ability to produce a moisture impervious seal. For each sample, two 6-inch square layers of material were bonded together using a 20 kHz ultrasonic rotary horn along the edges of the perimeters of the layers, approximately 0.1 inch from the edges. The material was a laminate of 0.6 osy polypropylene spunbond adhesively laminated to 0.6 mil polypropylene film, with the film sides of each of the two layers facing one another. The bond points in each of the patterns were round, each having a diameter of 0.045 inch (0.11 cm). The bond pattern sections along each 6-inch edge were arranged in four rows of bond points. The linear distance between adjacent bond points in a row, i.e., the machine direction spacing, ranged from 0.05 inch (0.13 cm) to 0.11 inch (0.28 cm), with actual values listed in Table 1. The vertical distance between horizontally offset bond points, i.e., the cross direction spacing, ranged from 0.10 (0.25 cm) to 0.30 (0.76 cm), with actual values listed in Table 1. The pattern width of the bond point sections, i.e., the distance between the first row of bond points and the fourth row of bond points, ranged from 0.210 inch to 0.270 inch, with actual values listed in Table 1. The bond points in adjacent rows were approximately 35 to 58 degrees offset from one another, with actual values listed in Table 1.

Before sealing the complete perimeter of the two layers, the resulting container was filled with 250 milliliters of blue-dyed saline solution. The bonded container was then set on a horizontal surface for 14 days and was observed for leakage. As can be seen in Table 1, the only tested bond pattern section that leaked was the section having machine direction spacing of 0.11 inch, cross direction spacing of 0.010 inch, and a pattern width of 0.210 inch.

**Table 1: Bond Point Sections Tested for Leakage**

| | | | | | |
|---|---|---|---|---|---|
| Sample | Machine Direction Space Between Bond Points (inches) | Cross Direction Space Between Bond Points (inches) | Pattern Width (inches) | Bond Angle (degrees) | Moisture Impervious |
| 1 | 0.05 | 0.010 | 0.210 | 35 | Yes |
| 2 | 0.05 | 0.020 | 0.240 | 40 | Yes |
| 3 | 0.05 | 0.030 | 0.270 | 44 | Yes |
| 4 | 0.08 | 0.010 | 0.210 | 41 | Yes |
| 5 | 0.08 | 0.020 | 0.240 | 46 | Yes |
| 6 | 0.08 | 0.030 | 0.270 | 50 | Yes |
| 7 | 0.11 | 0.010 | 0.210 | 49 | No |
| 8 | 0.11 | 0.020 | 0.240 | 54 | Yes |
| 9 | 0.11 | 0.030 | 0.270 | 58 | Yes |

The sample with the longest machine direction spacing (0.011 inch) and the shortest cross direction spacing (0.010 inch) was the only sample that leaked. The short cross direction spacing results in a small pattern width. The small pattern width translates to a short path through which fluid must travel to pass through the point bond section. It is believed that a point bond section having a combination of relatively long machine direction spacing and a relatively small pattern width results in a greater likelihood of leakage than a point bond section having relatively short machine direction spacing and/or a relatively large pattern width.

While the embodiments of the invention described herein are presently preferred, various modifications and improvements can be made without departing from the scope of the invention. The scope of the invention is indicated in the appended claims, and all changes that fall within the meaning and range of equivalents are intended to be embraced therein.

## Claims

1. A leak-proof seal (16), comprising:
at least two layers of liquid-impermeable material (12, 14) at least partially positioned in overlapping relationship;
a plurality of ultrasonic bond points (10) bonding together the at least two layers of liquid-impermeable material;
wherein the bond points are aligned in at least three parallel rows (18) and the bond points in adjacent rows are offset from one another, wherein each bond point is within 0.001 inch (0.03 mm) to 0.20 inch (5.1 mm) from at least one other bond point.

2. The leak-proof seal of Claim 1 wherein adjacent bond points in each row are at a distance of between 0.001 inch (0.03 mm) and 0.20 inch (5.1 mm) from one another.

3. The leak-proof seal of Claim 1 wherein adjacent bond points in each row are at a distance of between 0.0025 inch (0.064 mm) and 0.175 inch (4.45 mm) from one another.

4. The leak-proof seal of Claim 1 wherein adjacent bond points in each row are at a distance of between 0.005 inch (0.1 mm) and 0.15 inch (3.8 mm) from one another.

5. The leak-proof seal of Claim 1 wherein each bond point is within 0.0025 inch (0.064 mm) to 0.175 inch (4.45 mm) of at least one other bond point.

6. The leak-proof seal of Claim 1 wherein each bond point is within 0.005 inch (0.1 mm) and 0.15 inch (3.8 mm) of at least one other bond point.

7. The leak-proof seal of any preceding claim wherein each bond point has a diameter of between 0.005 inch (0.1 mm) and 0.25 inch (6.4 mm).

8. The leak-proof seal of Claim 7 wherein each bond point has a diameter of between 0.010 inch (0.25 mm) and 0.175 inch (4.45 mm).

9. The leak-proof seal of Claim 7 wherein each bond point has a diameter of between 0.015 inch (0.38 mm) and 0.15 inch (3.8 mm).

10. The leak-proof seal of any preceding claim wherein the bond points in adjacent rows are offset from one another by 0 degrees to 89 degrees.

11. The leak-proof seal of Claim 10 wherein the bond points in adjacent rows are offset from one another by 15 degrees to 75 degrees.

12. The leak-proof seal of Claim 10 wherein the bond points in adjacent rows are offset from one another by 30 degrees to 60 degrees.

13. The leak-proof seal of any preceding claim further comprising at least four parallel rows of bond points.

14. The leak-proof seal of any preceding claim wherein at least one of the layers of liquid-impermeable material is breathable.

15. The leak-proof seal of any preceding claim, wherein one of the at least two layers of liquid-impermeable material is a liquid-impermeable nonwoven laminate which comprises a liquid-impermeable film.

16. The leak-proof seal of any of claims 1 to 14 wherein at least one of the layers of liquid-impermeable material comprises a nonwoven web.

17. The leak-proof seal of any preceding claim, wherein the at least two layers are bonded along an edge of one of the layers.

18. A combination of a first liquid-impermeable, nonwoven laminate bonded to a second liquid-impermeable material, comprising:
the leak-proof seal of any preceding claim;
the first liquid-impermeable, nonwoven laminate and the second liquid-impermeable material being said at least two layers of liquid-impermeable material at least partially positioned in overlapping relationship.

19. The combination of Claim 18 wherein the first liquid-impermeable, nonwoven laminate comprises a liquid-impermeable film.

20. The combination of any of Claims 18 or 19 wherein the first liquid-impermeable, nonwoven laminate is breathable.

21. The combination of any of Claims 18, 19 or 20 wherein the second liquid-impermeable material comprises a nonwoven material laminated to a liquid-impermeable film.

22. The combination of any of Claims 18 to 21, wherein the second liquid-impermeable material is breathable.

23. A combination of a first liquid-impermeable material bonded to a second liquid-impermeable material, comprising:
the leak-proof seal of any of claims 1 to 17;
the first liquid-impermeable material and the second liquid-impermeable material being said at least two layers of liquid-impermeable material positioned in overlapping relationship; wherein
a garment comprises said second liquid-impermeable material and a containment flap comprises said first liquid-impermeable material.

24. An absorbent garment comprising a seal or combination of any preceding claim.

25. The garment of claim 24, wherein the garment is selected from the group consisting of a diaper, a training pant and an absorbent underpant.

26. A feminine hygiene article comprising a seal or combination of any of Claims 1 to 22.

27. An absorbent garment comprising a first liquid-impermeable material;
a containment flap comprising a second liquid-impermeable material; and
the leak-proof seal of any of claims 1 to 17;
said first liquid-impermeable material and said second liquid-impermeable material being said at least two layers of liquid-impermeable material at least partially positioned in overlapping relationship.

28. A bonding process for bonding together two layers of liquid-impermeable material to form the leak-proof seal of any of claims 1 to 13, comprising the steps of:
ultrasonically bonding the two layers together with discrete bond points aligned in at least three parallel rows, wherein the bond points are aligned in a pattern such that each bond point is within 0.001 inch (0.03 mm) to 0.20 inch (5.1 mm) of at least one other bond point, with the bond points in adjacent rows offset from one another; and
wherein the ultrasonic bonding comprises displacing portions (20) of each layer of material, wherein each of the displaced portions is in contact with at least one other displaced portion.

## Patentansprüche

1. Flüssigkeitsundurchlässige Dichtung (16), enthaltend:
wenigstens zwei Lagen aus flüssigkeitsundurchlässigem Material (12, 14), die einander wenigstens teilweise überlappend angeordnet sind;
mehrere Ultraschallschweißpunkte (10), die die wenigstens zwei Lagen aus flüssigkeitsundurchlässigem Material miteinander verschweißen;
wobei die Schweißpunkte in wenigstens drei parallelen Reihen (18) ausgerichtet sind und die Schweißpunkte in benachbarten Reihen gegeneinander versetzt sind, wobei jeder Schweißpunkt innerhalb von 0,01 Zoll (0,03 mm) bis 0,20 Zoll (5,1 mm) von wenigstens einem anderen Schweißpunkt entfernt liegt.

2. Flüssigkeitsundurchlässige Dichtung nach Anspruch 1, bei der benachbarte Schweißpunkte in jeder Reihe einen Abstand voneinander zwischen 0,001 Zoll (0,03 mm) und 0,20 Zoll (5,1 mm) haben.

3. Flüssigkeitsundurchlässige Dichtung nach Anspruch 1, bei der benachbarte Schweißpunkte in jeder Reihe einen Abstand voneinander zwischen 0,0025 Zoll (0,064 mm) und 0,175 Zoll (4,45 mm) haben.

4. Flüssigkeitsundurchlässige Dichtung nach Anspruch 1, bei der benachbarte Schweißpunkte in jeder Reihe einen Abstand voneinander zwischen 0, 005 Zoll (0,1 mm) und 0, 15 Zoll (3,8 mm) haben.

5. Flüssigkeitsundurchlässige Dichtung nach Anspruch 1, bei der jeder Schweißpunkt innerhalb von 0,0025 Zoll (0,064 mm) bis 0,175 Zoll (4,45 mm) von wenigstens einem weiteren Schweißpunkt liegt.

6. Flüssigkeitsundurchlässige Dichtung nach Anspruch 1, bei der jeder Schweißpunkt innerhalb von 0,005 Zoll (0,1 mm) bis 0,15 Zoll (3,8 mm) von wenigstens einem weiteren Schweißpunkt liegt.

7. Flüssigkeitsundurchlässige Dichtung nach einem der vorhergehenden Ansprüche, bei der jeder Schweißpunkt einen Durchmesser zwischen 0,005 Zoll (0,1 mm) und 0,25 Zoll (6,4 mm) hat.

8. Flüssigkeitsundurchlässige Dichtung nach Anspruch 7, bei der jeder Schweißpunkt einen Durchmesser zwischen 0,010 Zoll (0,25 mm) und 0,175 Zoll (4,45 mm) hat.

9. Flüssigkeitsundurchlässige Dichtung nach Anspruch 7, bei der jeder Schweißpunkt einen Durchmesser zwischen 0,015 Zoll (0,38 mm) und 0, 15 Zoll (3,8 mm) hat.

10. Flüssigkeitsundurchlässige Dichtung nach einem der vorhergehenden Ansprüche, bei der die Schweißpunkte in benachbarten Reihen gegeneinander um zwischen 0° und 89° versetzt sind.

11. Flüssigkeitsdurchlässige Dichtung nach Anspruch 10, bei der die Schweißpunkte in benachbarten Reihen gegeneinander um 15° bis 75° versetzt sind.

12. Flüssigkeitsdurchlässige Dichtung nach Anspruch 10, bei der die Schweißpunkte in benachbarten Reihen gegeneinander um 30° bis 60° versetzt sind.

13. Flüssigkeitsundurchlässige Dichtung nach einem der vorhergehenden Ansprüche, weiterhin enthaltend wenigstens vier parallele Reihen Schweißpunkte.

14. Flüssigkeitsundurchlässige Dichtung nach einem der vorhergehenden Ansprüche, bei der wenigstens eine der Lagen aus flüssigkeitsundurchlässigem Material atmungsaktiv ist.

15. Flüssigkeitsundurchlässige Dichtung nach einem der vorhergehenden Ansprüche, bei der eine der wenigstens zwei Lagen aus flüssigkeitsundurchlässigem Material ein flüssigkeitsundurchlässiges Vlieslaminat ist, das einen flüssigkeitsundurchlässigen Film enthält.

16. Flüssigkeitsundurchlässige Dichtung nach einem der Ansprüche 1 bis 14, bei der wenigstens eine der Lagen aus flüssigkeitsundurchlässigem Material eine Vliesbahn enthält.

17. Flüssigkeitsundurchlässige Dichtung nach einem der vorhergehenden Ansprüche, bei der wenigstens zwei Lagen längs eines Randes einer der Lagen miteinander verschweißt sind.

18. Kombination aus einem ersten flüssigkeitsundurchlässigen Vlieslaminat, das mit einem zweiten flüssigkeitsundurchlässigen Material verschweißt ist, enthaltend:
eine flüssigkeitsundurchlässige Dichtung nach einem der vorhergehenden Ansprüche;
das erste flüssigkeitsundurchlässige Vlieslaminat und das zweite flüssigkeitsundurchlässige Material, die die wenigstens zwei Lagen aus flüssigkeitsundurchlässigem Material sind, die wenigstens teilweise einander überlappend angeordnet sind.

19. Kombination nach Anspruch 18, bei der das erste flüssigkeitsundurchlässige Vlieslaminat einen flüssigkeitsundurchlässigen Film enthält.

20. Kombination nach einem der Ansprüche 18 oder 19, bei der das erste flüssigkeitsundurchlässige Vlieslaminat atmungsaktiv ist.

21. Kombination nach einem der Ansprüche 18, 19 oder 20, bei der das zweite flüssigkeitsundurchlässige Material ein Vliesmaterial enthält, das auf einen flüssigkeitsundurchlässigen Film laminiert ist.

22. Kombination nach einem der Ansprüche 18 bis 21, bei der das zweite flüssigkeitsundurchlässige Material atmungsaktiv ist.

23. Kombination eines ersten flüssigkeitsundurchlässigen Materials, das mit einem zweiten flüssigkeitsundurchlässigen Material verschweißt ist, enthaltend:
die flüssigkeitsundurchlässige Dichtung nach einem der Ansprüche 1 bis 17;
das erste flüssigkeitsundurchlässige Material und das zweite flüssigkeitsundurchlässige Material, die die wenigstens zwei Lagen aus flüssigkeitsundurchlässigem Material sind, die einander überlappend angeordnet sind; wobei
ein Kleidungsstück das zweite flüssigkeitsundurchlässige Material enthält und eine Behältnisklappe das erste flüssigkeitsundurchlässige Material enthält.

24. Ein absorbierendes Kleidungsstück, enthaltend eine Dichtung oder eine Kombination nach einem der vorhergehenden Ansprüche.

25. Das Kleidungsstück nach Anspruch 24, bei dem das Kleidungsstück aus der Gruppe ausgewählt ist, die aus einer Windel, einer Trainingshose und einer absorbierenden Unterhose besteht.

26. Ein Damenhygieneartikel, enthaltend eine Dichtung oder eine Kombination nach einem der Ansprüche 1 bis 22.

27. Ein absorbierendes Kleidungsstück, enthaltend ein erstes flüssigkeitsundurchlässiges Material;
eine Behältnisklappe, die ein zweites flüssigkeitsundurchlässiges Material enthält; und
eine flüssigkeitsundurchlässige Dichtung nach einem der Ansprüche 1 bis 17;
wobei das erste flüssigkeitsundurchlässige Material und das zweite flüssigkeitsundurchlässige Material die wenigstens zwei Lagen aus flüssigkeitsundurchlässigem Material sind, die einander wenigstens teilweise überlappend angeordnet sind.

28. Schweißvorgang zum Verschweißen zweier Lagen aus flüssigkeitsundurchlässigem Material zur Bildung einer flüssigkeitsundurchlässigen Dichtung nach einem der Ansprüche 1 bis 13, umfassend die folgenden Schritte:
Ultraschallverschweißung der zwei Lagen miteinander an diskreten Schweißpunkten, die in wenigstens drei parallelen Reihen ausgerichtet sind, wobei die Schweißpunkte in einem Muster derart ausgerichtet sind, dass jeder Schweißpunkt innerhalb von 0,001 Zoll (0,03 mm) bis 0,20 Zoll (5,1 mm) zu wenigstens einem weiteren Schweißpunkt liegt, wobei die Schweißpunkte in benachbarten Reihen gegeneinander versetzt sind; und
wobei die Ultraschallverschweißung das Verdrängen von Abschnitten (20) einer jeden Materiallage umfasst, wobei jeder der verdrängten Abschnitte mit wenigstens einem weiteren verdrängten Abschnitt in Berührung gelangt.

## Revendications

1. Fermeture étanche (16) comprenant :
au moins deux couches de matériau imperméable aux liquides (12, 14) positionnées au moins partiellement selon une relation de chevauchement ;
une pluralité de points de liaison ultrasonique (10) réunissant les au-moins-deux couches de matériau imperméable aux liquides ;
les points de liaison étant alignés selon au moins trois rangées (18) parallèles et les points de liaison, dans des rangées adjacentes, étant décalés les uns par rapport aux autres, chaque point de liaison étant à une distance comprise entre 0,001 pouce (0,03 mm) et 0,20 pouce (5,1 mm) d'au moins un autre point de liaison.

2. Fermeture étanche selon la revendication 1, dans laquelle, dans chaque rangée, des points de liaison adjacents sont espacés les uns des autres d'une distance comprise entre 0,001 pouce (0,03 mm) et 0,20 pouce (5,1 mm).

3. Fermeture étanche selon la revendication 1, dans laquelle, dans chaque rangée, des points de liaison adjacents sont espacés les uns des autres d'une distance comprise entre 0,0025 pouce (0,064 mm) et 0,175 pouce (4,45 mm).

4. Fermeture étanche selon la revendication 1, dans laquelle, dans chaque rangée, des points de liaison adjacents sont espacés les uns des autres d'une distance comprise entre 0,005 pouce (0,1 mm) et 0,15 pouce (3,8 mm).

5. Fermeture étanche selon la revendication 1, dans laquelle chaque point de liaison est à une distance comprise entre 0,0025 pouce (0,064 mm) et 0,175 pouce (4,45 mm) d'au moins un autre point de liaison.

6. Fermeture étanche selon la revendication 1, dans laquelle chaque point de liaison est à une distance comprise entre 0,005 pouce (0,1 mm) et 0,15 pouce (3,8 mm) d'au moins un autre point de liaison.

7. Fermeture étanche selon l'une quelconque des revendications précédentes, dans laquelle chaque point de liaison a un diamètre compris entre 0,005 pouce (0,1 mm) et 0,25 pouce (6,4 mm).

8. Fermeture étanche selon la revendication 7, dans laquelle chaque point de liaison a un diamètre compris entre 0,010 pouce (0,25 mm) et 0,175 pouce (4,45 mm).

9. Fermeture étanche selon la revendication 7, dans laquelle chaque point de liaison a un diamètre compris entre 0,015 pouce (0,38 mm) et 0,15 pouce (3,8 mm).

10. Fermeture étanche selon l'une quelconque des revendications précédentes, dans laquelle, dans des rangées adjacentes, les points de liaison sont décalés les uns par rapport aux autres de 0 degré à 89 degrés.

11. Fermeture étanche selon la revendication 10, dans laquelle, dans des rangées adjacentes, les points de liaison sont décalés les uns par rapport aux autres de 15 degrés à 75 degrés.

12. Fermeture étanche selon la revendication 10, dans laquelle, dans les rangées adjacentes, les points de liaison sont décalés les uns par rapport aux autres de 30 degrés à 60 degrés.

13. Fermeture étanche selon l'une quelconque des revendications précédentes, comprenant, en outre, au moins quatre rangées parallèles de points de liaison.

14. Fermeture étanche selon l'une quelconque des revendications précédentes, dans laquelle l'une au moins des couches de matériau imperméable aux liquides est respirante.

15. Fermeture étanche selon l'une quelconque des revendications précédentes, dans laquelle l'une des au-moins-deux couches de matériau imperméable aux liquides est un laminé non-tissé imperméable aux liquides comprenant un film imperméable aux liquides.

16. Fermeture étanche selon l'une quelconque des revendications 1 à 14, dans laquelle l'une au moins des couches de matériau imperméable aux liquides comprend un voile non-tissé.

17. Fermeture étanche selon l'une quelconque des revendications précédentes, dans laquelle les au-moins-deux couches sont liées le long d'un bord de l'une des couches.

18. Combinaison d'un premier laminé non-tissé, imperméable aux liquides, lié à un second matériau imperméable aux liquides, comprenant :
la fermeture étanche selon l'une quelconque des revendications précédentes ;
le premier laminé non-tissé, imperméable aux liquides et le second matériau imperméable aux liquides étant lesdites au-moins-deux couches de matériau imperméable aux liquides positionnées au moins partiellement selon une relation de chevauchement.

19. Combinaison selon la revendication 18, dans laquelle le premier laminé non-tissé, imperméable aux liquides, comprend un film imperméable aux liquides.

20. Combinaison selon l'une quelconque des revendications 18 ou 19, dans laquelle le premier laminé non-tissé, imperméable aux liquides, est respirant.

21. Combinaison selon l'une quelconque des revendications 18, 19 ou 20, dans laquelle le second matériau imperméable aux liquides comprend un matériau non-tissé laminé à un film imperméable aux liquides.

22. Combinaison selon l'une quelconque des revendications 18 à 21, dans laquelle le second matériau imperméable aux liquides est respirant.

23. Combinaison d'un premier matériau imperméable aux liquides lié à un second matériau imperméable aux liquides, comprenant :
la fermeture étanche selon l'une quelconque des revendications 1 à 17 ;
le premier matériau imperméable aux liquides et le second matériau imperméable aux liquides étant lesdites au-moins-deux couches de matériau imperméable aux liquides positionnées selon une relation de chevauchement ;
un vêtement comprenant ledit second matériau imperméable aux liquides et un volet de confinement comprenant ledit premier matériau imperméable aux liquides.

24. Vêtement absorbant comprenant une fermeture étanche ou une combinaison selon l'une quelconque des revendications précédentes.

25. Vêtement selon la revendication 24, dans lequel le vêtement est sélectionné dans le groupe consistant en un change pour nourrisson, une culotte d'apprentissage de la propreté et un sous-vêtement absorbant.

26. Article d'hygiène féminine comprenant une fermeture étanche ou une combinaison selon l'une quelconque des revendications 1 à 22.

27. Vêtement absorbant comprenant un premier matériau imperméable aux liquides ;
un volet de confinement comprenant un second matériau imperméable aux liquides ; et
la fermeture étanche selon l'une quelconque des revendications 1 à 17 ;
ledit premier matériau imperméable aux liquides et ledit second matériau imperméable aux liquides étant lesdites au-moins-deux couches de matériau imperméable aux liquides positionnées au moins partiellement selon une relation de chevauchement.

28. Procédé de liaison destiné à la réunion de deux couches de matériau imperméable aux liquides pour former la fermeture étanche selon l'une quelconque des revendications 1 à 13, comprenant les étapes suivantes :
la réunion par liaison ultrasonique des deux couches au moyen de points de liaison discrets, alignés selon au moins trois rangées parallèles, les points de liaison étant alignés selon un motif tel que chaque point de liaison est à une distance comprise entre 0,001 pouce (0,03 mm) et 0,20 pouce (5,1 mm) d'au moins un autre point de liaison, les points de liaison, dans des rangées adjacentes, étant décalés les uns par rapport aux autres ; et
la liaison ultrasonique comprend le déplacement de portions (20) de chaque couche de matériau, chacune des portions déplacées étant en contact avec au moins une autre portion déplacée.
